(19) Europäisches Patentamt
European Patent Office
Office européen des brevets

(11) EP 0 678 580 B1

(12) **EUROPEAN PATENT SPECIFICATION**

(45) Date of publication and mention of the grant of the patent:
**21.02.2007 Bulletin 2007/08**

(51) Int Cl.:
***C12Q 1/26*** *(2006.01)* ***G01N 33/72*** *(2006.01)*

(21) Application number: **94309842.6**

(22) Date of filing: **28.12.1994**

(54) **Reagent for measuring direct bilirubin**

Reagens zur Messung von direktem Bilirubin

Reactif pour la mesure de bilirubine directe

(84) Designated Contracting States:
**DE FR GB IT**

(30) Priority: **28.12.1993 JP 33866193**
**27.10.1994 JP 26419794**

(43) Date of publication of application:
**25.10.1995 Bulletin 1995/43**

(73) Proprietors:
- **UNITIKA LTD.**
  **Amagasaki-shi**
  **Hyogo (JP)**
- **Mitsubishi Kagaku Iatron, Inc.**
  **Shinjuku-ku, Tokyo (JP)**

(72) Inventors:
- **Senba,Shoji**
  **c/o Unitika Ltd.,**
  **Uji-shi**
  **Kyoto (JP)**
- **Kurosaka,Keisuke**
  **c/o Unitika Ltd.,**
  **Kyoto (JP)**
- **Kondo,Hitoshi**
  **c/o Unitika Ltd.,**
  **Kyoto (JP)**
- **Kojima,Masami**
  **c/o cen Res Lab,**
  **Iatron Lab,Inc.**
  **Katori-gun**
  **Chiba-ken (JP)**
- **Suzuki,Hiroshi**
  **c/o Yachiyo Plant,**
  **Yachiyo-shi**
  **Chiba-ken (JP)**

(74) Representative: **Towler, Philip Dean**
**Frank B. Dehn & Co.**
**St Bride's House**
**10 Salisbury Square**
**London EC4Y 8JD (GB)**

(56) References cited:
**EP-A- 0 098 562** **EP-A- 0 114 381**
**EP-A- 0 484 133**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 678 580 B1

## Description

FIELD OF THE INVENTION

The present invention relates to reagents for measuring direct bilirubin present in biological fluids.

PRIOR ART

Bilirubin is a yellow pigment belonging to tetrapyrroles. It is a degradative product of heme, and is known to be present in bile in large quantities. Bilirubin in biological fluids is mainly composed of direct and indirect bilirubin.

The amount of indirect bilirubin increases significantly as a result of hemolytic anemina and jaundice, while the amount of direct bilirubin increases in obstructive jaundice. For this reason, separate determination for direct and indirect bilirubin is important in clinical diagnosis. Total and direct bilirubin have conventionally been measured in clinical laboratories. However, a more accurate method to determine the amount of direct bilirubin is demanded.

Various methods are available for determining the amount of direct bilirubin, including methods such as the ones which use diazo regents, methods which use high performance liquid chromatography(HPLC), methods which use oxidases such as bilirubin oxidase, and methods which use oxidizing agents such as vanadic acid ions and manganic ions.

Various methods using diazoreagents which use different diazotizing accelerators and different methods of determining the azobilirubin produced, have been reported so far. The reagent reported by Malloy and Evelyn [Journal of Biological Chemistry, Vol. 119, p481 (1937)] is one. United States Patent No. 4,892,833 discloses a process and reagent kit for the determination of direct and total bilirubin in body fluids by coupling the bilirubin with a diazonium salt and determining the extinction change thereby brought about.

Methods which use HPLC to determine the amount of bilirubin include, for example, a method which uses phosphate buffers and isopropanol as an eluent for reversed phase HPLC column, reported by Lauff et al [Journal of Chromatography, Vol. 226, p391 (1981)].

In methods which use oxidases, bilirubin is oxidized by the oxidases, which cause the disappearance of its yellow color at around a wavelength of 450 nm. The change in absorbance of the bilirubin solution is then measured before and after the reaction, to obtain the amount of bilirubin. It is possible to specifically oxidize direct bilirubin by varying reaction conditions. Reagents used in such methods include, for example, reagents containing bilirubin oxidase [Clinical Chemistry, Vol. 20, p783 (1979)] and reagents containing lactase, tyrosinase, ascorbate oxidase and other oxidases (Japanese patent 62-33880). Further, reagents for measuring direct bilirubin with bilirubin oxidases optimized to react only with direct bilirubin, by suitably selecting pH, buffers, and surfactants, have been proposed.

These reagents for measuring direct bilirubin include, for example, reagents containing bilirubin oxidase, which are reacted in buffers of pH 9 to 11 (Japanese patent 5-68240), reagents containing bilirubin oxidases in acid buffers of pH 5 to 6 containing anionic surfactants (Japanese patent 5-9066), reagents containing bilirubin oxidases in buffers of pH 3.5 to 4.5 (Japanese patent 61-44000), reagents containing bilirubin oxidases in buffers of pH 2.0 to 3.3 with potassium ferrocyanide and/or pottasium ferricyanide (Japanese application patent 1-5499).

In methods using oxidizing agents, bilirubin is oxidized by oxidizing agents, which cause the disappearance of its yellow color at around a wavelength of 450 nm, and the change in absorbance of the bilirubin solution is measured before and after the reaction to determine the amount of bilirubin. It is also possible to specifically oxidize direct bilirubin by varying reaction conditions. The reagents used in these methods include, for example, reagents using vanadic acid ions [Clinical Chemistry, Vol. 22, p116 (1993)]. Further, reagents for the measurement of direct bilirubin, in which oxidizing agents react only with direct bilirubin, by suitably selecting inhibitors, buffers, etc., to inhibit the reaction of indirect bilirubin, have been proposed. These reagents for measuring direct bilirubin include, for example, a reagent for determining the amount of direct bilirubin by using vanadic acid ions or manganic ions as oxidizing agents, and one or more compounds from hydrazines, hydroxylamines, oximes, aliphatic polyvalent amines, phenols, water-soluble macromolecules and nonionic surfactants with HLB value 15 or above, as reaction inhibitors for indirect bilirubin (Japanese patent application 5-18978).

Conventional methods to differentially determine the amount of direct bilirubin by using HPLC have been reported to sufficiently separate direct bilirubin from other bilirubin species, but have a number of problems, such as the fact that they use expensive and special equipment, take a long time to analyze, and can not analyze a number of specimens at a time.

On the other hand, the methods to determine the amount of direct bilirubin by allowing bilirubin oxidases or other oxidases to react have advantages such as the possibility of using optical measurements, the simplicity of the method, and its relatively high precision. However, they do not have sufficient ability for the separation of bilirubins, because the reaction with direct bilirubin is not perfect, or they react with not only direct but also some indirect bilirubin. In addition, these reactions must be carried out outside the optimum pH range for bilirubin oxidases. For the above reasons, a large amount of enzymes must be used and the reagents become expensive. To solve such problems, the inventors of the present invention improved specificity for direct bilirubin by adding fluorides and/or reducing agents and proposed reagents for measuring direct bilirubin that can be used in the optimum pH range of BOD (Japanese patent application 5-276992). It should be noted, however, that the ability to separate direct and indirect bilirubin for this reagent is not perfect.

Reagents using vanadic acid ions or manganic ions as oxidizing agents will react with not only direct but also some indirect bilirubin, resulting in insufficient fractional determination even when inhibitors for indirect bilirubin such as hydrazines, hydroxylamines, oximes, etc. are used.

The methods using diazo reagents are designed for the separation of direct and indirect bilirubins, in which diazotizing reaction accelerators are or are not added. The problem with using this type of reagent is its imperfection in separating the two bilirubins, just like the reagents with oxidases and oxidizing agents. The imperfection is particularly conspicuous when measuring specimens of low albumin content and specimens containing salicylic acids and other medicines.

SUMMARY OF THE INVENTION

The inventors of the present invention have been studying means to solve the problems of the conventional reagents for measuring direct bilirubin, taking in consideration the above-mentioned circumstances. As a result, the present invention was completed by finding the fact that direct bilirubin can be measured with good precision without reaction of indirect bilirubin by allowing tetrapyrrole compounds to exist in the reagents. Such reagents may be reagents which measure direct bilirubin that specifically oxidize direct bilirubin, by bilirubin oxidases, oxidizing agents, or by converting direct bilirubin into azobilirubin by the reaction of diazonium salts.

More specifically, the present invention provide a reagent for measuring direct bilirubin which allows the optical measurement of the absorbance change of direct bilirubin, resulting from the reaction of bilirubin oxidases, wherein tetrapyrrole compounds are added to the reagent with bilirubin oxidase.

Further, the present invention provides a reagent for measuring direct bilirubin which allows the optical measurement of the absorbance change in direct bilirubin, resulting from its reaction with an oxidizing agent, wherein tetrapyrrole compounds are added to the reagent with oxidizing agents.

Still further, the present invention provides a reagent for measuring direct bilirubin which allows the optical measurement of the absorbance change in direct bilirubin resulting from its reaction with diazonium salts, wherein tetrapyrrole compounds are added to the reagent with diazonium salts.

BRIEF DESCRIPTION OF THE DRAWINGS

Figure 1 shows the relationship between the measured value of the amount of direct bilirubin and the dilution ratio of samples containing indirect bilirubin. The reagent used is that of the present invention which comprises bilirubin and enzymes (bilirubin oxidase) derived from Trachyderma (for Example 1) or Myrothecium (for Example 2).

For Comparative Examples 1 and 2, the same samples were measured using reagents without bilirubin, and are also shown in the figure.

Figure 2 shows the relationship between the measured value of the amount of direct bilirubin and the dilution ratio of samples containing indirect bilirubin. The reagent used is that of the present invention which comprises bilirubin and enzymes (bilirubin oxidase) derived from Myrothecium. Comparative examples in which the reagents used contain no tetrapyrrole compound, are also shown in the figure.

Figure 3 shows the relationship between the measured value of the amount of direct bilirubin and the dilution ratio of samples which contain direct bilirubin. The reagent used is that of the present invention, comprising bilirubin and enzymes (bilirubin oxidase) derived from Trachyderma.

Figure 4 shows the relationship between the measured value of the amount of direct bilirubin and the dilution ratio of samples which contain direct bilirubin. The reagent used is that of the present invention comprising bilirubin and enzymes (bilirubin oxidase) derived from Myrothecium.

Figure 5 shows the relationship between the measured value of the amount of direct bilirubin and the dilution ratio of samples which contain direct bilirubin. The reagent used is that of the present invention comprising bilirubin and enzymes (bilirubin oxidase) derived from Myrothecium.

Figure 6 shows the relationship between the measured value of the amount of direct bilirubin and the dilution ratio of samples which contain direct bilirubin. The reagent used is that of the present invention comprising bilirubin and enzymes (bilirubin oxidase) derived from Myrothecium.

Figure 7 shows the relationship between the measured value of the amount of direct bilirubin and the dilution ratio of samples which contain direct bilirubin, for Examples 21 to 24. The reagent used is that of the present invention, which contains Sodium Metavanadate (as an oxidizing agent).

Figure 8 shows the relationship between the measured value of the amount of direct bilirubin and the dilution ratio of samples which contain direct bilirubin for Examples 25 to 28. The reagent used is that of the present indention, with sulfanic acid (as a diazo compound).

Figure 9 shows the relationship between the measured value of direct bilirubin and the dilution ratio of samples containing direct bilirubin, for Examples 29 to 32. The reagent is that of the present invention, containing sulfanic acid (as a diazo compound).

DETAILED DESCRIPTION OF THE INVENTION

The reagent for measuring direct bilirubin according to the present invention may be a two-stage reagent system comprising a first reagent containing a buffer solution and tetrapyrrole compound(s), and a second reagent containing bilirubin oxidases, oxidizing agents, or diazonium salts; or a single reagent system in which all components are contained in one solution.

The tetrapyrrole compounds added to the reagents are not particularly limited in the present invention. For example, bilirubin, biliverdin, and urobilinogen may be used independently or in combination. Other tetrapyrrole compounds may be chosen, as well. The use of bilirubin is most preferable. The concentration may be in the range of 0.01 to 100 μg/ml, preferably 0.1 to 40 μg/ml. If the concentration of the tetrapyrrole compounds is below the lower limit of the above range, indirect bilirubin is partially oxidized with direct bilirubin, which is not desirable.

Bilirubin oxidases used in the reagent of the present invention may be, for example, enzymes derived from Myrothecium or Trachyderma. The amount required is 0.001 to 200 units/ml, more preferably 0.005 to 20 units/ml.

The buffer to be used should have a buffering capacity of pH 6.5 or less, preferably between pH 3.0 to 6.5, which is favorable for the enzymes to be used from the viewpoint of their stability and activity. Examples include phthalic acid-sodium hydroxide buffer, malic acid-sodium hydroxide buffer, citric acid-sodium citrate buffer, etc. The concentration of the buffer may be 20 to 500 mM, preferably 30 to 300 mM.

Other components may be adequately added such as p-toluenesulfonic acid, benzoic acid and other aromatic carboxylic acids, surfactants, alanines, serines and other amino acids, mannitol and other sugars, polyethylene glycol and other polyols, NaCl and other salts, albumin and other proteins, etc.

The type of vanadic acid ions that may be used as oxidizing agents in the measuring reagent of the present invention are not particularly limited. Favorable examples include those containing $VO_3^-$, $VO_4^{3-}$ or other 5 valence vanadiums. Although vanadic acids may be used alone, it is preferable to use vanadic acids in the form of salts of lithium, sodium, potassium and other alkaline metals, or ammonium, from the viewpoint of solubility.

Manganic ions may be used as oxidizing agent in the measuring reagent of the present invention, and may not only be ordinary manganic salts such as manganese (III) acetate but also compounds that form chelates with acetylacetone, ethylenediaminetetraacetic acid or other chelating agents.

The concentration of vanadic acid ions and manganese ions is not particularly limited provided that it is sufficient to allow oxidation of bilirubin contained in the specimen. It should generally be in the range of 0.1 to 50 μg ions/ml, preferably 0.5 to 10 μg ions/ml.

Hydrazines, hydroxylamines, oximes, aliphatic polyvalent amines, phenols, water soluble macromolecules, nonionic surfactants of HLB value 15 or above, may be used as inhibitors for indirect bilirubin in the reagent of the present invention. The type of these inhibitors are not particularly limited, provided that the oxidation of direct bilirubin is not affected. Preferable examples for each type are as follows: Hydrazine species include, for example, hydrazines, phenylhydrazines and their mineral acid salts (hydrochloric acid salts, sulfate, etc.). Hydroxylamine species include, for example, hydroxylamines, phenylhydroxylamines and their mineral acid salts (hydrochloric acid salts, sulfate, etc.). Oxime species include, for example, acetoxime, diacetylmonooxime, salicylaldoxime, etc. Aliphatic polyvalent amine species include, for example, tetraethylenepentamine hexamethylene-tetramine, triethylenetetramine, etc. Phenol species include, for example, phenol, p-chlorphenol, p-acetamidephenol, 4-chlor-1-naphthol, β-naphthol, etc. Water soluble macromolecules include, for example, polyvinylalcohol, polyvinylpyrrolidone, etc. Nonionic surfactants of HLB value 15 or above include, for example, Emulgen-123p (polyoxy-ethylenelaurylether, by Kao), Emulgen-950 (polyoxyethylene-nonylphenylether, by Kao), Emulgen-985 (polyoxyethylene-nonylphenylether, by Kao), Triton X-405 (polyoxyethylene-isooctylphenylether, by Rohm and Haas Co.), etc. These inhibitors are effective for inhibiting the oxidation of indirect bilirubin independently. The effect may be enhanced when two or more inhibitors are used in combination. These inhibitors are adequately selected for each application. The concentration of these inhibitors is not particularly limited provided that they can inhibit the oxidation of indirect bilirubin in the specimen, and may depend on the type of inhibitor chosen, but is generally in the 0.01 to 10% (W/V) range.

In the reagent of the present invention, the reagent blank value decreases, leading to an improvement in the precision of the analysis, when chelating agents are added. Also, the reagent is stabilized, and the oxidation of bilirubin is accelerated with the addition of chelating agents. Favorable chelating agents that may be used for the above purpose include, for example, ethylenediaminetetraacetic acid (EDTA), nitrirotriacetic acid (NTA), cyclohexanediamine-tetra-acetic acid (CyDTA), diethylene-triamineOpenta-acetic acid (DTPA), hydroxyethyl ethylenediamine-tri-acetic acid (EDTA-OH), tri-ethylene-tetraimine-hexa-acetic acid (TTHA), hydroxyethylimine-di-acetic acid (HIDA), 1-hydroxyethane-1, 1-diphosphoric acid or their alkaline metal salts (for example, lithium salts, sodium salts, potassium salts, etc.) and ammonia salts, etc. The concentration of these chelating agents is not particularly limited provided that the measurement of bilirubin is not affected, and may be in the range of 0.02 to 65 mM, preferably 1 to 40 mM, more preferably between 1 and 25 mM.

Diazo reagents used in the reagent of the present invention may be any known diazo reagent used in conventional methods, such as, for example, unstabilized diazo reagents disclosed in the Japanese application patent 56-12555 (diazotized derivatives of sulfanilic acid, o-dianisidine, p-chloroaniline, 1,5-dichloroaniline, 2,4-dichloroaniline, 2-methoxy-4-nitroaniline, 1-aminoanthraquinone, 2-nitroaniline, and 4-chloromethylaniline) and stabilized diazo reagents (borate tetrafluoride, 1,5-naphthalenedisulfonic acid salts and zinc chloride salts of the above-mentioned unstabilized diazo reagents). In particular, 2,4-dichlorphenyl diazonium-1,5-naphthalenedisulfonic acid and its salts (for example, sodium salts, potassium salts, etc.), p-sulfanylbenzene diazonium-1,5-naphthalenesulfonic acid and its salts, p-nitrobenzene diazonium-tetra fluoride boric acid and its salts, diazo-sulfanyl acid-tetra-fluoride boric acid and its salts, and other stabilized diazonium salts allow simple measurement and thus are effective diazo reagents to be used in the reagent of the present invention.

Other reagents such as buffers and antiseptics, may be appropriately selected from buffers used in known chemical oxidation methods, bilirubin oxidase methods, etc. (for example, phosphate, citrate, succinate, acetate, phthalate etc.) and from antiseptics such as paraben and used in the reagent of the present invention. Their concentration and the pH value of the measuring reagents may also be appropriately selected according to those specified for reagents in said known methods.

The reagent of the present invention may also be prepared by appropriately selecting the above-mentioned constituents and mixing them according to known methods. A favorable example comprises a first reagent containing 10 to 200 mM phthalate buffer adjusted to pH 4.7 to 6.5, 0.005 to 0.5% polyethylene-glycomono-p-isooctylphenylether, 5 to 100 mM alanine, 1 to 100 mM p-toluenesulfonic acid, 0.001 to 1% human serum albumin, 0.05 to 200 mM sodium fluoride, 0.02 to 10 mM N-acetylcysteine, and 0.1 to 10 $\mu$g/ml bilirubin, and a second reagent containing 0.001 to 100 units/ml bilirubin oxidase and 10 to 200 mM phthalate buffer adjusted to pH 4.7 to 6.5. It is possible to prepare reagents that allow a more accurate measurement of the concentration of direct bilirubin, by adding 1 to 10 $\mu$g/l bilirubin to a first reagent of commercially available diagnostical reagents, for measuring direct bilirubin, such as NESCAUTO D-BIL-VE (Nippon Shoji Kaisha Ltd.), Ekdia DB (EIKEN Chemical), or D-BIL reagent C (International Reagents Corp.), etc.

Direct bilirubin may be measured by the method of the present invention described in detail below, by using the reagents of the present invention.

First, for example, to a solution containing a buffer and bilirubin or other tetrapyrrole compounds, an adequate amount of various specimens containing bilirubin (blood plasma, serum, urine or other biological fluids) is added. The solution is then pre-heated in the cell of a spectrophotometer and the absorbance is measured at a specified wavelength for the solution (460 nm or thereabout) (absorbance 1). A second reagent containing bilirubin oxidase is added to the solution, and reaction is allowed to take place for 1 to 10 minutes for direct bilirubin to be oxidized. The absorbance is measured again at 460 nm or thereabout (absorbance 2). The change in absorbance (A) at the above-mentioned wavelength is determined by multiplying a dilution factor of the reagent to absorbance 1 and 2. Then, a standard substance containing direct bilirubin of a known concentration is measured by the same procedures to obtain the change in absorbance (B), and to obtain a calibration curve. The change in absorbance (A) is then overlaid on the calibration curve to determine the amount of direct bilirubin present in the specimen. Using the change in absorbance (A) and (B), the direct bilirubin content of the specimen is calculated using the following equation.

Concentration of direct bilirubin in specimen (mg/dl)
= A/B x concentration of direct bilirubin in standard solution (mg/dl)

Volume of the specimen may preferably be 0.005 to 0.1 ml. The wavelength at which measurement is performed is not limited to 460 nm but may be any wavelength in the 400 to 480 nm range.
The volume of the first and the second reagent and the specimen may vary appropriately.

The reaction may be run under normal conditions. For example, the reaction temperature may be in the range of 25 to 45 degC, preferably 35 to 40 degC, and the reaction time may be in the range of 1 to 30 minutes, preferably 3 to 15 minutes.

When measuring the amount of direct bilirubin using an oxidising agent, a specimen containing bilirubin is added to a first reagent comprising a buffer inhibitor and, when required, chelating agents, to create a solution. Absorbance of the solution is measured at a specific wavelength (between 430 and 460 nm) (absorbance 1). Then a second reagent comprising vanadic acid ions or manganic ions and, when required, chelating agents, is added to the above solution. Oxidation of bilirubin occurs at 25 to 40 degC for 3 to 15 minutes. The absorbance at the specific wavelength is then measured again (absorbance 2). The change in absorbance (A) at the above-mentioned specific wavelength is determined by multiplying a dilution factor of the reagent to absorbance 1 and 2. Then, the change in absorbance (A) is overlaid on a calibration curve, obtained from the change in absorbance (B) for the reaction of a standard substance, to determine the amount of direct bilirubin in the specimen. Alternatively, the above equation is used to calculate the amount of the direct bilirubin in the specimen.

The same procedure may be applied when using a diazonium salt. A buffer is used as the first reagent, a specimen containing bilirubin is added to the buffer to make a solution, and the absorbance of the solution is measured at a specific wavelength (between 540 and 600 nm) (absorbance 1). Then, the second reagent containing diazonium salts is added to the above solution and reacted at 25 to 40 degC for 3 to 15 minutes. The absorbance at the specific wavelength is measured again (absorbance 2). The change in absorbance (A) is determined from absorbance (1) and (2), and the amount of direct bilirubin in the specimen is measured from the change in absorbance (B) which is measured beforehand. An alternative method of measuring the concentration of direct bilirubin using a diazonium salt is described below.

The first reagent containing diazonium salts is mixed with a specimen containing bilirubin. The mixture reacts at 25 to 40 degC for 3 to 15 minutes. A buffer is added to the solution. Further reaction takes place at 25 to 40 degC for 3 to 15 minutes.

The absorbance of this solution is measured at a specific wavelength (between 540 and 660 nm). Then, the absorbance is overlaid on the calibration curve derived from the absorbance obtained from the reaction of a standard substance, to measure the amount of the direct bilirubin in the specimen.

Other measuring methods are also available. For example, in the above method which uses an oxidising agent, the absorbance of the solution comprising a mixture of the first reagent and the specimen is taken as absorbance 1 and the absorbance of the solution comprising a mixture of the first and the second reagent, pre-mixed, which is allowed to react with the specimen, is taken as absorbance 2. Using these two measured values, the concentration of direct bilirubin in the specimen is determined by following the above procedures.

As described above, the reagents of the present invention do not react with indirect bilirubin existing in bilirubin-containing specimens because the reagents of the present invention contain tetrapyrrole compounds together with bilirubin oxidases, vanadic acids or manganic ions, or diazo reagent. It is thus possible to measure the amount of direct bilirubin alone with good precision.

## Examples

The present invention is further illustrated by the following examples which, however, should not be construed as limiting the invention or its details. Absorbance was measured for the examples using a Hitachi 7070 automatic analyzer.

<Preparation of Specimen>

Specimen 1: Bilirubin (by Sigma) was dissolved in 100 mM tris-hydrochloric acid buffer (pH 8.0). 0, 0.2, 0.4, 0.6, 0.8 and 1.0 mg/dl bilirubin solutions containing bovine serum albumin was added to give a specimen of indirect bilirubin. In other words, the concentrations of direct bilirubin in these specimens were Omg/dl. The concentration of the bovine serum albumin (by Boehringer Mannheim Japan) solution used was 3.2 g/dl. The pH of tris-hydrochloric acid buffer was 8.5.

Specimen 2: Using the bovine serum albumin solution of Specimen 1, and Ortho liquid normal control serum (by Ortho Diagnostics Corp., hereafter abbreviated "Ortho normal"), a specimen of Ortho normal (without bilirubin) was prepared.

Specimen 3: 0.5 mg/dl of bilirubin was added to Ortho normal. Specimen 4: 1.0 mg/dl of bilirubin was added to Ortho normal. Specimen 5: A direct bilirubin specimen was prepared by diluting High Level Check BIL (by Takara Shuzo Co., Ltd.) solution with physiological saline.

<Preparation of Reagents>

Reagent 1: Reagent 1 was prepared by adding 60 mM Potassium-hydrogen-phthalate (pH 5.0), 0.01% polyethyleneglycolmono-p-isooctylphenylether, 0.002% human serum albumin, 40 mM p-toluenesulfonic acid, 2.0 mM N-acetylcysteine, 2.0 mM sodium fluoride, 40 mM alanine, and 0.5 μg/ml bilirubin.
Reagent 2: Reagent 2 was prepared by adding 5.0 units/ml bilirubin oxidase derived from Trachyderma (by Takara Shuzo Co., Ltd.) to Reagent 1.
Reagent 3: Reagent 3 was prepared by adding 5 μg/ml bilirubin to the first reagent of commercially available diagnostic regent kit, a reagent for measuring direct bilirubin known as NESCAUTO D-BIL-VE (by Nippon Shoji Kaisha Ltd.).
Reagent 4: The second reagent of NESCAUTO D-BIL-VE (by Nippon Shoji Kaisha Ltd.) was used as Reagent 4.
Reagent 5: Reagent 5 was prepared by adding 5 μg/ml bilirubin to the first reagent of Ekdia DB (by EIKEN Chemical).
Reagent 6: The second reagent of Ekdia DB (by EIKEN Chemical) was used as Reagent 6.
Reagent 7: Reagent 7 was prepared by adding 5 μg/ml bilirubin to the first reagent of D-BIL reagent C (by International Reagents Corp.).
Reagent 8: The second reagent of D-BIL reagent C (International Reagents Corp.).
Reagents 4, 6 and 8 contain bilirubin oxidases derived from Myrothecium (by Amano Pharmaceutical Co., Ltd.)
Reagent 9: Reagent 1 without bilirubin.
Reagent 10: Reagent 3 without bilirubin.
Reagent 11: Reagent 5 without bilirubin.
Reagent 12: Reagent 7 without bilirubin.
Reagent 13: 20 mM hydrochloric acid hydroxylamine and 10 mM hydroxyethanedisulfonic acid were added to 100 mM tartaric acid buffer of pH 2.9.
Reagent 14 : 0.4 μg/ml bilirubin was added to Reagent 13.
Reagent 15: 4 μg/ml bilirubin was added to Reagent 13.
Reagent 16: 20 μg/ml bilirubin was added to Reagent 13.
Reagent 17: 40 μg/ml bilirubin was added to Reagent 13.
Reagent 18: 8 mM hydroxyethanedisulfonic acid and 4 mM Sodium Metavanadate were added to a phosphate buffer of pH 7.0.
Reagent 19: 65 mM hydrochloric acid of pH 1.0.
Reagent 20: 0.4 μg/ml bilirubin was added to Reagent 19.
Reagent 21: 4 μg/ml bilirubin was added to Reagent 19.
Reagent 22: 20 μg/ml bilirubin was added to Reagent 19.
Reagent 23: 40 μg/ml bilirubin was added to Reagent 19.
Reagent 24: A reagent comprising 8.1 mM sulfanilic acid and 1.4 mM sodium nitrite was prepared.
Reagent 25: A reagent comprising 5.1 mM sulfanilic acid and 0.5mM sodium nitrite, (blue color under the alkaline condition) was prepared.
Reagent 26: 0.4 μg/ml bilirubin was added to Reagent 25.
Reagent 27: 4 μg/ml bilirubin was added to Reagent 25.
Reagent 28: 20 μg/ml bilirubin was added to Reagent 25.
Reagent 29: 40 μg/ml bilirubin was added to Reagent 25.
Reagent 30: 22 mM tartaric acid buffer adjusted to pH 13.5.

<Example 1>

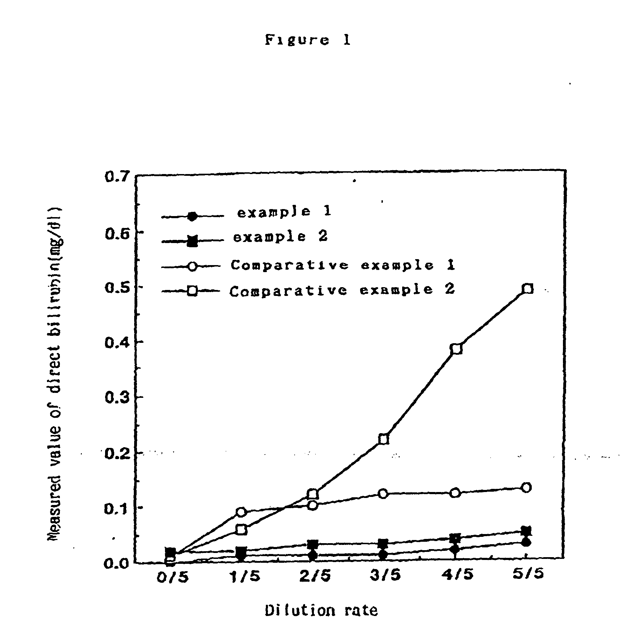

Specimen 1 (0.014 ml) was added to the above Reagent 1 (0.28 ml). The mixture was heated at 37 degC for 5 minutes. Sample blanks were corrected according to the final volume by measuring the absorbance of the solution at 450 nm. Reagent 2 (0.07 ml) was added and the solution was allowed to react at 37 deg C for 5 minutes. The absorbance of the solution at 450 nm was measured and the amount of direct bilirubin in the specimen was determined using ditaurobilirubin of known concentration as the standard. The result is shown in Figure 1.

<Comparative Example 1>

The same measurement as that in example 1 was performed using Reagent 9 in place of Reagent 1.
The results of the above examples are shown in Figure 1.

<Example 2, Comparative Example 2>

A sample was prepared through the same procedure as that taken in Example 1, using Reagents 3 and 4 instead of Reagents 1 and

2. (Example 2)

A sample was prepared through the same procedure as that taken in Comparative Example 1, using Reagent 10 instead of Reagent 9. (Comparative Example 2)

The amount of direct bilirubin in the samples were determined by measuring their absorbance at 450 nm. The results are shown in Figure 1.

<Examples 3 and 4, Comparative Examples 3 and 4>

A sample was prepared through the same procedure as that taken in Example 1, using Reagents 5 and 6 (for Example 3), and Reagent 7 and 8 (for Example 9), instead of Reagents 1 and 2.

A sample was prepared through the same procedure as that taken in Comparative example 1, using Reagent 11 (for Comparative Example 3) and Reagent 12 (for Comparative Example 4) instead of Reagent 9.

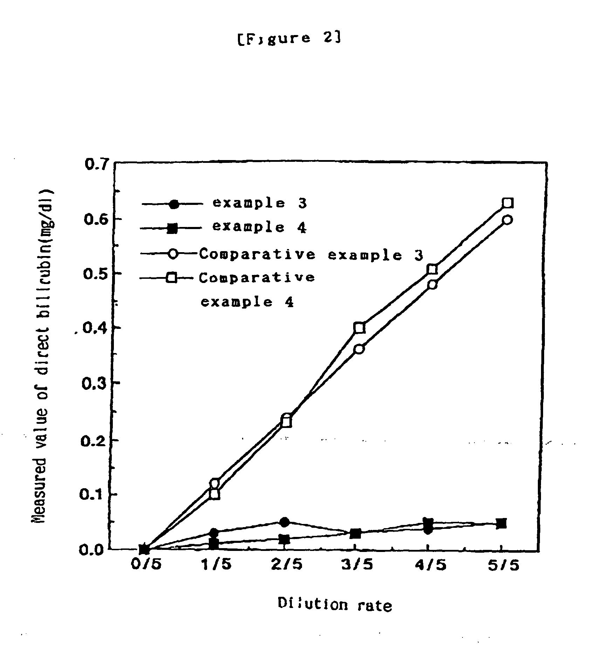

The amount of direct bilirubin in the samples prepared for Examples 3,4 and Comparative Examples 3, 4 were determined by measuring the absorbance of each solution at 450 nm. The results are shown in Figure 2.

These results indicate that indirect bilirubin is not oxidized by the reagent of the present invention.

<Examples 5, 6, 7, and 8>

A sample was prepared through the same procedure as that taken in Example 1, 2, 3, and 4 except that specimen 1 (which contains indirect bilirubin) was replaced by specimen 5 (which contains direct bilirubin).

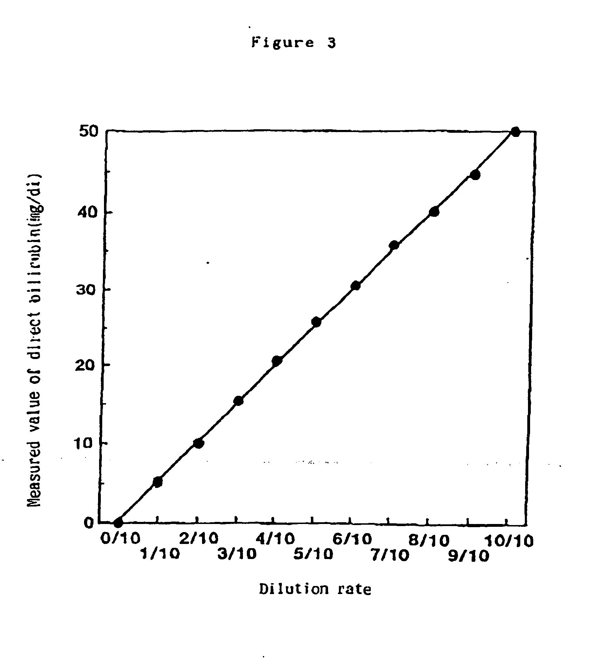

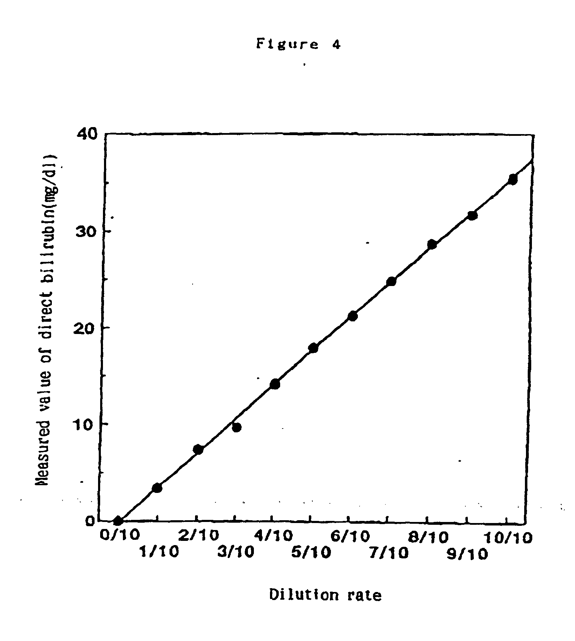

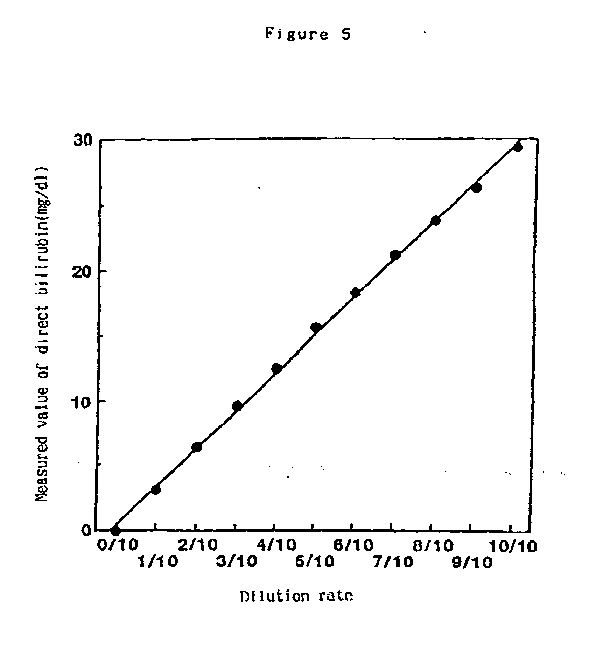

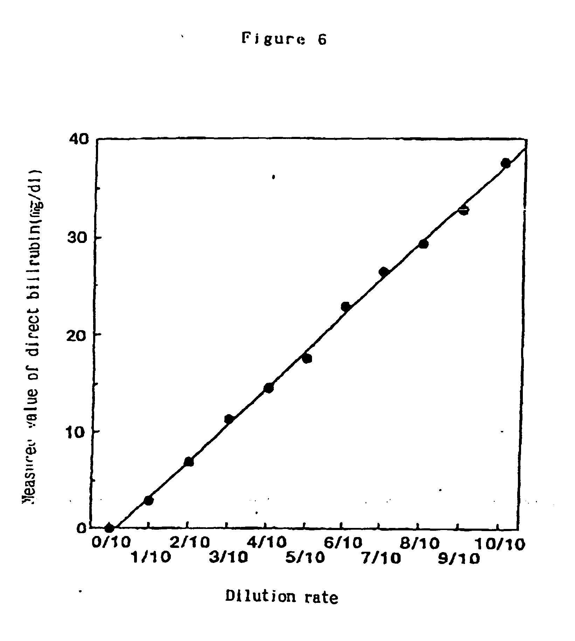

The amount of direct bilirubin in the samples prepared were determined by measuring the absorbance of each solution at 450 nm. The relationship between the measured values for each sample and the concentration of specimen 5, are shown in Figure 3, 4, 5, and 6, respectively.

<Example 9>

Specimen 2, 3, or 4 was added to Reagent 14 (0.28 ml). The mixture was heated at 37 C for 5 minutes. The absorbance of the mixture was measured at 450 nm (subwavelength 546 nm), with the sample blank corrected for the final volume of fluid. Using a ditaurobilirubin as a standard, the amount of direct bilirubin was determined. Then, Reagent 18 (0.07 ml) was added. The mixture was heated at 37 C for 5 minutes, and the absorbance of the mixture was measured at 450 nm (subwavelength 546 nm), with the sample blank corrected for the final volume of fluid.

<Examples 10, 11, and 12>

A sample was prepared as described in Example 9, using Reagent 15 (for Example 10), Reagent 16 (for Example 11), and Reagent 17 (for Example 12) instead of Reagent 14. The same procedure was taken for these samples as in Example 9.

<Comparative Example 5>

Specimen 2, 3, or 4 was added to Reagent 13 (0.28 ml). The mixture was treated with the same procedures as in Example 9.

The amount of direct bilirubin determined for Examples 9 to 12 and Comparative Example 5, are shown in Table 1. The results indicate that the indirect bilirubin is not oxidized by the reagent of the present invention.

[Table 1]

| | Specimen 2 | Specimen 3 | Specimen 4 |
|---|---|---|---|
| comparative Example 5 | 0.36 | 0.53 | 0.70 |
| Working Example 9 | 0.38 | 0.43 | 0.48 |
| Working Example 10 | 0.34 | 0.35 | 0.37 |
| Working Example 11 | 0.13 | 0.18 | 0.15 |

(continued)

| | Specimen 2 | Specimen 3 | Specimen 4 |
|---|---|---|---|
| Working Example 12 | 0.06 | 0.03 | 0.04 |

(mg/dl)

<Example 13>

Specimen 2, 3, or 4 was added to 0.3 ml of Reagent 20, and heated for 37 C for 5 minutes. The absorbance of the sample at 546 nm (sub-wavelength 660 nm) was corrected according to the final volume of the sample. Then reagent 24 (0,15 mL) was added to the mixture. The mixture was allowed to react at 37 C for 5 minutes, and the absorbance was measured at 546 (sub-wavelenght 660 nm). Then, the amount of direct bilirubin was obtained from the measured absorbance of each sample at 546 nm (sub-wavelength 660 nm), using a ditaurobilirubin solution of known concentration as a standard.

<Examples 14, 15 and 16>

The above procedure of Example 13 was performed using Reagents 21 (for Example 14), 22 (for Example 15), and 23 (for Example 16) instead of Reagent 20.

<Comparative Example 6>

Specimen 2, 3, or 4 was added to 0.3 ml of Reagent 19, and heated for 37 C for 5 minutes. The absorbance of the sample at 546 nm (sub-wavelength 660 nm) was corrected according to the final volume of the sample. Then, the amount of direct bilirubin was obtained from the measured absorbance of each sample at 546 nm (sub-wavelength 660 nm), using a ditaurobilirubin solution of known concentration as a standard.
The results of Examples 13 to 16 and Comparative Example 6 are shown in Table 2.
These results suggest that indirect bilirubin is not oxidized by the Reagent of the present invention.

[Table 2]

| | Specimen 2 | Specimen 3 | Specimen 4 |
|---|---|---|---|
| Comparative Example 6 | 0.32 | 0.55 | 0.78 |
| Working Example 13 | 0.34 | 0.45 | 0.52 |
| Working Example 14 | 0.45 | 0.41 | 0.45 |
| Working Example 15 | 0.39 | 0.37 | 0.35 |
| Working Example 16 | 0.40 | 0.41 | 0.41 |

(mg/dl)

<Example 17>

Specimen 2, 3, or 4 (0.01 ml each) were added to 0.15 ml of Reagent 26, and heated for 37 C for 5 minutes. Then, 0.3 ml of Reagent 30 was added, and heated at 37 C for another 5 minutes. The amount of direct bilirubin was obtained from the measured absorbance of each sample at 600 nm (sub-wavelength 660 nm), using a ditaurobilirubin solution of known concentration as a standard.

<Examples 18, 19, and 20>

The above procedure of Example 17 was performed using Reagents 27 (for Example 18), 28 (for Example 19), and 29 (for Example 20) instead of Reagent 26.

<Comparative Example 7>

Specimen 2, 3, or 4 (0.01 ml each) were added to 0.15 ml of Reagent 25, and heated for 37 C for 5 minutes. Then, 0.3 ml of Reagent 30 was added, and heated at 37 C for another 5 minutes. The amount of direct bilirubin was obtained from the measured absorbance of each sample at 600 nm (sub-wavelength 660 nm), using a ditaurobilirubin solution of known concentration as a standard. The results obtained by the above Examples (17 to 20) and Comparative Example 7 are shown in Table 3.
These results indicate that the Reagent of the present invention does not oxidize indirect bilirubin.

[Table 3]

| | Specimen 2 | Specimen 3 | Specimen 4 |
|---|---|---|---|
| Comparative Example 7 | 0.35 | 0.51 | 0.71 |
| Working Example 17 | 0.27 | 0.38 | 0.49 |
| Working Example 18 | 0.39 | 0.43 | 0.43 |
| Working Example 19 | 0.31 | 0.32 | 0.35 |
| Working Example 20 | 0.14 | 0.19 | 0.19 |

(mg/dl)

<Examples 21 to 32>

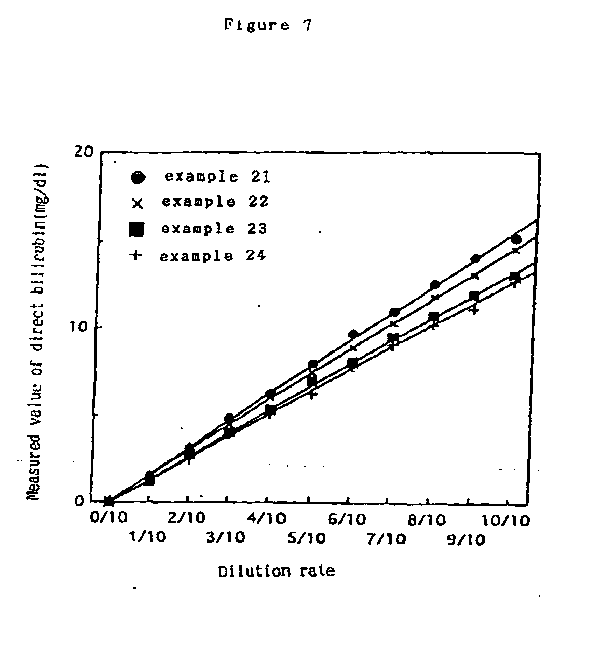

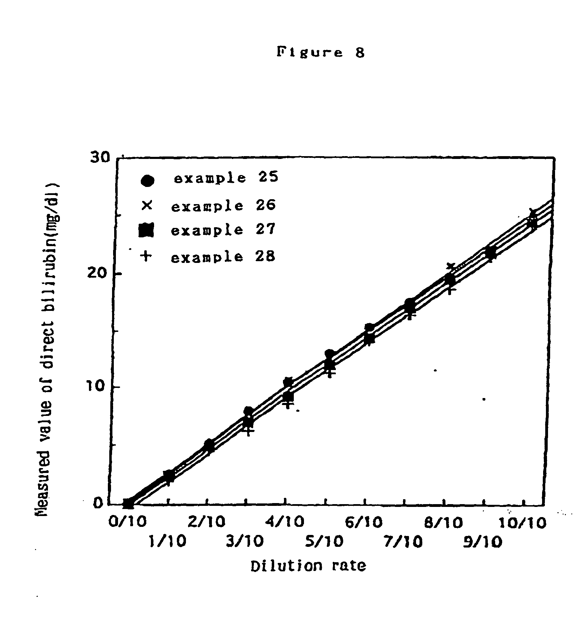

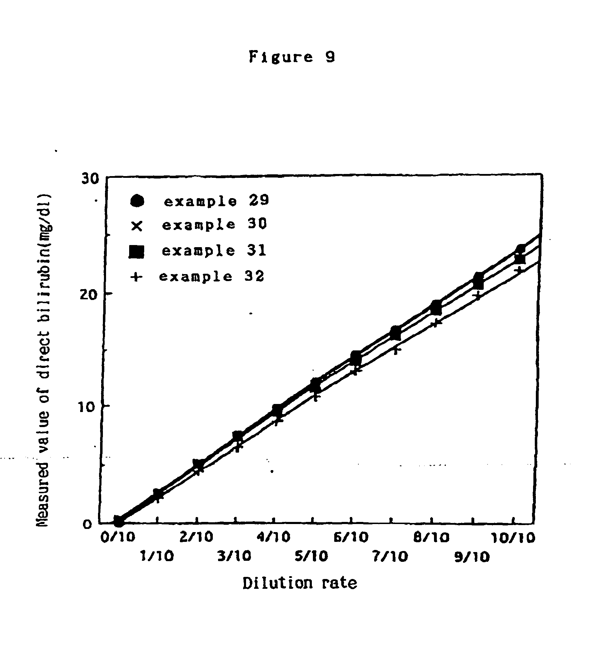

The measurements of Examples 9, 10, 11, and 12, Examples 13, 14, 15, and 16, and Examples 17, 18, 19, and 20 were performed using Specimen 5 instead of Specimen 1. These measurements are numbered Examples 21, 22, 23, and 24, Examples 25, 26, 27, and 28, and Examples 29, 30, 31, and 32, consecutively.
The results of Examples 21 to 24 are shown in Figure 7.
The results of Examples 25 to 28 are shown in Figure 8.
The results of Examples 29 to 32 are shown in Figure 9.
These Figures show the relationship between the ratio of dilution of the Reagents, and the measured value of the concentration of direct bilirubin in each samples. The fact that the results obtained show a straight line that passes through a point close to the origin, indicates that the Reagent and Method of the present invention give accurate results for testing direct bilirubin in a range sufficient for their use in actual clinical tests.

As described in detail, the present invention allows the measurement of direct bilirubin, which is known to increase significantly in biological fluids of patients with obstructive jaundice, separately from indirect bilirubin, and is useful in clinical and other examinations, where accuracy is demanded.

**Claims**

1. A reagent for measuring direct bilirubin in a specimen, said reagent comprising one or more tetrapyrrole compound and an entity selected from the group consisting of a bilirubin oxidase, an oxidizing agent, and a diazonium salt.

2. A reagent according to claim 1 which also comprises a buffer.

3. A reagent according to claim 1 or claim 2, wherein the one or more tetrapyrrole compound is selected from the group consisting of bilirubin, biliverdin, urobilinogen, and combinations thereof.

4. A reagent according to any of claims 1 to 3, wherein the tetrapyrrole compound is present at a concentration of 0.1 to 40 μg/ml.

5. A reagent according to claim 2, which comprises a first component comprising a buffer and one or more tetrapyrrole compound, and a second component comprising an entity selected from the group consisting of a bilirubin oxidase, an oxidizing agent, and a diazonium salt.

6. A reagent according to claim 5, wherein the tetrapyrrole compound is selected from the group consisting of bilirubin, biliverdin, urobilinogen, and combinations thereof.

7. A reagent according to claim 5 or claim 6, wherein the tetrapyrrole compound is present at a concentration of 0.1 to 40 μg/ml.

8. A method for optically measuring direct bilirubin in a specimen, which comprises the addition of one or more tetrapyrrole compound, and an entity selected from the group consisting of a bilirubin oxidase, an oxidizing agent, and a diazonium salt, to a specimen, and the measurement of the resulting change in absorbance.

**9.** A method according to claim 8 wherein the tetrapyrrole compound is bilirubin.

**10.** A method according to claim 8 or claim 9 comprising the steps of:

a. combining the specimen with a buffer and a tetrapyrrole compound to form a first solution thereof;
b. heating the first solution;
c. measuring the absorbance of the first solution at a specified wavelength;
d. adding an entity selected from the group consisting of a bilirubin oxidase, an oxidizing agent, and a diazonium salt to the first solution to form a second solution;
e. measuring the absorbance of the second solution at a specified wavelength, and determining a change in absorbance (A) between the absorbance of the first and second solutions;
f. performing the steps a-e on a standard sample containing direct bilirubin at a known concentration to determine a change in absorbance (B) for the standard sample; and
g. relating A, B and the known concentration of direct bilirubin in the standard sample to determine the concentration of direct bilirubin in the specimen.

**11.** A method according to claim 8 or 9 comprising the steps of:

a. Adding a reagent comprising a buffer and one or more tetrapyrrole compound to a specimen;
b. Equilibrating this mixture at a chosen temperature in a spectrophotometer;
c. Measuring the absorbance at a particular wavelength between 400 and 480 nm;
d. Adding a reagent comprising a bilirubin oxidase.
e. Allowing the reaction to occur for 1 to 30 minutes at 25 to 45°C;.
f. Re-measuring the absorbance, calculating the change in absorbance and correcting this to allow for dilution;
g. Calculating the direct bilirubin content of the specimen by comparing its change in absorbance with that of standard samples.

**12.** A method according to claim 8 or 9 comprising the steps of:

a. Adding a reagent comprising a buffer and one or more tetrapyrrole compound to a specimen;
b. Equilibrating this mixture at a chosen temperature in a spectrophotometer;
c. Measuring the absorbance at a particular wavelength between 430 and 460 nm;
d. Adding a reagent comprising a manganic or vanadic oxidising agent;
e. Allowing the reaction to occur for 3 to 15 minutes at 25 to 40°C;
f. Re-measuring the absorbance, calculating the change in absorbance and correcting this to allow for dilution;
g. Calculating the direct bilirubin content of the specimen by comparing its change in absorbance with that of standard samples.

**13.** A method according to claim 8 or 9 comprising the steps of:

a. Adding a reagent comprising a buffer and one or more tetrapyrrole compound to a specimen;
b. Equilibrating this mixture at a chosen temperature in a spectrophotometer;
c. Measuring the absorbance at a particular wavelength between 540 and 600 nm;
d. Adding a reagent comprising a diazonium salt;
e. Allowing the reaction to occur for 3 to 15 minutes at 25 to 40°C;
f. Re-measuring the absorbance, calculating the change in absorbance and correcting this to allow for dilution;
g. Calculating the direct bilirubin content of the specimen by comparing its change in absorbance with that of standard samples.

**14.** Use of a reagent comprising one or more tetrapyrrole compound and an entity selected from the group consisting of a bilirubin oxidase, an oxidizing agent, and a diazonium salt for measuring direct bilirubin in a specimen.

**15.** Use according to claim 14 wherein the reagent also comprises a buffer.

**16.** Use according to claim 14 or claim 15, wherein the one or more tetrapyrrole compound is selected from the group consisting of bilirubin, biliverdin, urobilinogen, and combinations thereof.

**17.** Use according to any of claims 14 to 16, wherein the tetrapyrrole compound is present at a concentration of 0.1 to 40 μg/ml.

**18.** Use according to claim 15, wherein the reagent comprises a first component containing a buffer and one or more tetrapyrrole compound, and a second component comprising an entity selected from the group consisting of a bilirubin oxidase, an oxidizing agent, and a diazonium salt.

**19.** Use according to claim 18, wherein the tetrapyrrole compound is selected from the group consisting of bilirubin, biliverdin, urobilinogen, and combinations thereof.

**20.** Use according to claim 18 or claim 19, wherein the tetrapyrrole compound is present at a concentration of 0.1 to 40 μg/ml.

**Patentansprüche**

**1.** Ein Reagenz zur Messung von direktem Bilirubin in einer Probe, wobei das Reagenz eine oder mehrere Tetrapyrrolverbindung(en) und eine Einheit, ausgewählt aus der Gruppe, bestehend aus einer Bilirubin-Oxidase, einem Oxidationsmittel und einem Diazoniumsalz, enthält.

**2.** Ein Reagenz gemäß Anspruch 1, das auch einen Puffer enthält.

**3.** Ein Reagenz gemäß Anspruch 1 oder Anspruch 2, wobei die eine oder die mehreren Tetrapyrrolverbindung(en) ausgewählt ist/sind aus der Gruppe, bestehend aus Bilirubin, Biliverdin, Urobilinogen und Kombinationen davon.

**4.** Ein Reagenz gemäß irgendeinem der Ansprüche 1 bis 3, wobei die Tetrapyrrolverbindung in einer Konzentration von 0,1 bis 40 μg/ml vorliegt.

**5.** Ein Reagenz gemäß Anspruch 2, das eine erste Komponente, enthaltend einen Puffer und eine oder mehrere Tetrapyrrolverbindung(en), und eine zweite Komponente, enthaltend eine Einheit, ausgewählt aus der Gruppe, bestehend aus einer Bilirubin-Oxidase, einem Oxidationsmittel und einem Diazoniumsalz, enthält.

**6.** Ein Reagenz gemäß Anspruch 5, wobei die Tetrapyrrolverbindung ausgewählt ist aus der Gruppe, bestehend aus Bilirubin, Biliverdin, Urobilinogen und Kombinationen davon.

**7.** Ein Reagenz gemäß Anspruch 5 oder Anspruch 6, wobei die Tetrapyrrolverbindung in einer Konzentration von 0,1 bis 40 μg/ml vorliegt.

**8.** Ein Verfahren zur optischen Messung von direktem Bilirubin in einer Probe, das die Zugabe von einer oder mehreren Tetrapyrrolverbindung(en) und einer Einheit, ausgewählt aus der Gruppe, bestehend aus einer Bilirubin-Oxidase, einem Oxidationsmittel und einem Diazoniumsalz, zu einer Probe und die Messung der resultierenden Änderung der Extinktion umfasst.

**9.** Ein Verfahren gemäß Anspruch 8, bei dem die Tetrapyrrolverbindung Bilirubin ist.

**10.** Ein Verfahren gemäß Anspruch 8 oder Anspruch 9, umfassend die Schritte:

a. Kombinieren der Probe mit einem Puffer und einer Tetrapyrrolverbindung, um eine erste Lösung daraus zu bilden,
b. Erwärmen der ersten Lösung,
c. Messen der Extinktion der ersten Lösung bei einer vorgegebenen Wellenlänge,
d. Zugeben einer Einheit, ausgewählt aus der Gruppe, bestehend aus einer Bilirubin-Oxidase, einem Oxidationsmittel und einem Diazoniumsalz, zu der ersten Lösung, um eine zweite Lösung zu bilden,
e. Messen der Extinktion der zweiten Lösung bei einer vorgegebenen Wellenlänge und Ermitteln einer Änderung der Extinktion (A) zwischen der Extinktion der ersten und der zweiten Lösung,
f. Durchführen der Schritte a-e bei einer Standard-Probe, die direktes Bilirubin in einer bekannten Konzentration enthält, um eine Änderung der Extinktion (B) bei der Standard-Probe zu ermitteln, und
g. In-Beziehung-Setzen von A, B und der bekannten Konzentration von direktem Bilirubin in der Standard-Probe, um die Konzentration von direktem Bilirubin in der Probe zu ermitteln.

**11.** Ein Verfahren gemäß Anspruch 8 oder 9, umfassend die Schritte:

a. Zugeben eines Reagenzes, das einen Puffer und eine oder mehrere Tetrapyrrolverbindung(en) enthält, zu einer Probe,
b. Äquilibrieren dieser Mischung bei einer gewählten Temperatur in einem Spektrophotometer,
c. Messen der Extinktion bei einer bestimmten Wellenlänge zwischen 400 und 480 nm,
d. Zugeben eines Reagenzes, das eine Bilirubin-Oxidase enthält,
e. Ablaufenlassen der Reaktion 1 bis 30 Minuten lang bei 25 bis 45°C,
f. erneutes Messen der Extinktion, Berechnen der Änderung der Extinktion und Berichtigen dieser, um die Verdünnung zu berücksichtigen,
g. Berechnen des Gehalts der Probe an direktem Bilirubin durch Vergleich ihrer Änderung der Extinktion mit der von Standard-Proben.

**12.** Ein Verfahren gemäß Anspruch 8 oder 9, umfassend die Schritte:

a. Zugeben eines Reagenzes, das einen Puffer und eine oder mehrere Tetrapyrrolverbindung(en) enthält, zu einer Probe,
b. Äquilibrieren dieser Mischung bei einer gewählten Temperatur in einem Spektrophotometer,
c. Messen der Extinktion bei einer bestimmten Wellenlänge zwischen 430 und 460 nm,
d. Zugeben eines Reagenzes, das ein Mangan- oder Vanadium-Oxidationsmittel enthält,
e. Ablaufenlassen der Reaktion 3 bis 15 Minuten lang bei 25 bis 40°C,
f. erneutes Messen der Extinktion, Berechnen der Änderung der Extinktion und Berichtigen dieser, um die Verdünnung zu berücksichtigen,
g. Berechnen des Gehalts der Probe an direktem Bilirubin durch Vergleich ihrer Änderung der Extinktion mit der von Standard-Proben.

**13.** Ein Verfahren gemäß Anspruch 8 oder 9, umfassend die Schritte:

a. Zugeben eines Reagenzes, das einen Puffer und eine oder mehrere Tetrapyrrolverbindung(en) enthält, zu einer Probe,
b. Äquilibrieren dieser Mischung bei einer gewählten Temperatur in einem Spektrophotometer,
c. Messen der Extinktion bei einer bestimmten Wellenlänge zwischen 540 und 600 nm,
d. Zugeben eines Reagenzes, das ein Diazoniumsalz enthält,
e. Ablaufenlassen der Reaktion 3 bis 15 Minuten lang bei 25 bis 40°C,
f. erneutes Messen der Extinktion, Berechnen der Änderung der Extinktion und Berichtigen dieser, um die Verdünnung zu berücksichtigen,
g. Berechnen des Gehalts der Probe an direktem Bilirubin durch Vergleich ihrer Änderung der Extinktion mit der von Standard-Proben.

**14.** Verwendung eines Reagenzes, das eine oder mehrere Tetrapyrrolverbindung(en) und eine Einheit, ausgewählt aus der Gruppe, bestehend aus einer Bilirubin-Oxidase, einem Oxidationsmittel und einem Diazoniumsalz, enthält, zur Messung von direktem Bilirubin in einer Probe.

**15.** Verwendung gemäß Anspruch 14, wobei das Reagenz auch einen Puffer enthält.

**16.** Verwendung gemäß Anspruch 14 oder Anspruch 15, wobei die eine oder die mehreren Tetrapyrrolverbindung(en) ausgewählt ist/sind aus der Gruppe, bestehend aus Bilirubin, Biliverdin, Urobilinogen und Kombinationen davon.

**17.** Verwendung gemäß irgendeinem der Ansprüche 14 bis 16, wobei die Tetrapyrrolverbindung in einer Konzentration von 0,1 bis 40 µg/ml vorliegt.

**18.** Verwendung gemäß Anspruch 15, wobei das Reagenz eine erste Komponente, enthaltend einen Puffer und eine oder mehrere Tetrapyrrolverbindung(en), und eine zweite Komponente, enthaltend eine Einheit, ausgewählt aus der Gruppe, bestehend aus einer Bilirubin-Oxidase, einem Oxidationsmittel und einem Diazoniumsalz, enthält.

**19.** Verwendung gemäß Anspruch 18, wobei die Tetrapyrrolverbindung ausgewählt ist aus der Gruppe, bestehend aus Bilirubin, Biliverdin, Urobilinogen und Kombinationen davon.

**20.** Verwendung gemäß Anspruch 18 oder Anspruch 19, wobei die Tetrapyrrolverbindung in einer Konzentration von 0,1 bis 40 μg/ml vorliegt.

**Revendications**

**1.** Réactif pour la mesure de bilirubine conjuguée dans un spécimen, ledit réactif comprenant un ou plusieurs composés tétrapyrroliques et une entité choisie dans le groupe consistant en une bilirubine oxydase, un agent oxydant, et un sel de diazonium.

**2.** Réactif selon la revendication 1, qui comprend également un tampon.

**3.** Réactif selon la revendication 1 ou la revendication 2, dans lequel ledit un ou lesdits plusieurs composés tétrapyrroliques sont choisis dans le groupe consistant en bilirubine, biliverdine, urobilinogène, et des combinaisons de ceux-ci.

**4.** Réactif selon l'une quelconque des revendications 1 à 3, dans lequel le composé tétrapyrrolique est présent à une concentration de 0,1 à 40 μg/ml.

**5.** Réactif selon la revendication 2, qui comprend un premier composant comprenant un tampon et un ou plusieurs composés tétrapyrroliques, et un deuxième composant comprenant une entité choisie dans le groupe consistant en une bilirubine oxydase, un agent oxydant, et un sel de diazonium.

**6.** Réactif selon la revendication 5, dans lequel le composé tétrapyrrolique est choisi dans le groupe consistant en bilirubine, biliverdine, urobilinogène, et des combinaisons de ceux-ci.

**7.** Réactif selon la revendication 5 ou la revendication 6, dans lequel le composé tétrapyrrolique est présent à une concentration de 0,1 à 40 μg/ml.

**8.** Procédé pour la mesure optique de bilirubine conjuguée dans un spécimen, qui comprend l'addition d'un ou plusieurs composés tétrapyrroliques, et d'une entité choisie dans le groupe consistant en une bilirubine oxydase, un agent oxydant, et un sel de diazonium, à un spécimen, et la mesure de la variation résultante dans l'absorbance.

**9.** Procédé selon la revendication 8, dans lequel le composé tétrapyrrolique est la bilirubine.

**10.** Procédé selon la revendication 8 ou la revendication 9, comprenant les étapes consistant à :

a. combiner le spécimen à un tampon et un composé tétrapyrrolique pour former une première solution de ceux-ci ;
b. chauffer la première solution ;
c. mesurer l'absorbance de la première solution à une longueur d'ondes spécifiée ;
d. ajouter une entité choisie dans le groupe consistant en une bilirubine oxydase, un agent oxydant, et un sel de diazonium, à la première solution pour former une deuxième solution ;
e. mesurer l'absorbance de la deuxième solution à une longueur d'ondes spécifiée, et déterminer une variation dans l'absorbance (A) entre l'absorbance de la première solution et de la deuxième solution ;
f. exécuter les étapes a à e sur un échantillon de référence contenant la bilirubine conjuguée à une concentration connue pour déterminer une variation dans l'absorbance (B) pour l'échantillon de référence ; et
g. relier A, B et la concentration connue de bilirubine conjuguée dans l'échantillon de référence pour déterminer la concentration de bilirubine conjuguée dans le spécimen.

**11.** Procédé selon la revendication 8 ou la revendication 9, comprenant les étapes consistant à :

a. ajouter un réactif comprenant un tampon et un ou plusieurs composés tétrapyrroliques à un spécimen ;
b. équilibrer ce mélange à une température choisie dans un spectrophotomètre ;
c. mesurer l'absorbance à une longueur d'ondes particulière entre 400 et 480 nm ;
d. ajouter un réactif comprenant une bilirubine oxydase ;

e. laisser la réaction se dérouler pendant 1 à 30 minutes à 25 à 45 °C ;
f. re-mesurer l'absorbance, calculer la variation dans l'absorbance et corriger celle-ci pour permettre la dilution ;
g. calculer la teneur en bilirubine conjuguée du spécimen par comparaison de sa variation dans l'absorbance à celle des échantillons de référence.

**12.** Procédé selon la revendication 8 ou la revendication 9, comprenant les étapes consistant à :

a. ajouter un réactif comprenant un tampon et un ou plusieurs composés tétrapyrroliques à un spécimen ;
b. équilibrer ce mélange à une température choisie dans un spectrophotomètre ;
c. mesurer l'absorbance à une longueur d'ondes particulière entre 430 et 460 nm ;
d. ajouter un réactif comprenant un agent oxydant manganique ou vanadique ;
e. laisser la réaction se dérouler pendant 3 à 15 minutes à 25 à 40 °C ;
f. re-mesurer l'absorbance, calculer la variation dans l'absorbance et corriger celle-ci pour permettre la dilution ;
g. calculer la teneur en bilirubine conjuguée du spécimen par comparaison de sa variation dans l'absorbance à celle des échantillons de référence.

**13.** Procédé selon la revendication 8 ou la revendication 9, comprenant les étapes consistant à :

a. ajouter un réactif comprenant un tampon et un ou plusieurs composés tétrapyrroliques à un spécimen ;
b. équilibrer ce mélange à une température choisie dans un spectrophotomètre ;
c. mesurer l'absorbance à une longueur d'ondes particulière entre 540 et 600 nm ;
d. ajouter un réactif comprenant un sel de diazonium ;
e. laisser la réaction se dérouler pendant 3 à 15 minutes à 25 à 40 °C ;
f. re-mesurer l'absorbance, calculer la variation dans l'absorbance et corriger celle-ci pour permettre la dilution ;
g. calculer la teneur en bilirubine conjuguée du spécimen par comparaison de sa variation dans l'absorbance à celle des échantillons de référence.

**14.** Utilisation d'un réactif comprenant un ou plusieurs composés tétrapyrroliques et une entité choisie dans le groupe consistant en une bilirubine oxydase, un agent oxydant, et un sel de diazonium, pour la mesure de bilirubine directe dans un spécimen.

**15.** Utilisation selon la revendication 14, dans laquelle le réactif comprend également un tampon.

**16.** Utilisation selon la revendication 14 ou la revendication 15, dans laquelle ledit un ou lesdits plusieurs composés tétrapyrroliques sont choisis dans le groupe consistant en bilirubine, biliverdine, urobilinogène, et des combinaisons de ceux-ci.

**17.** Utilisation selon l'une quelconque des revendications 14 à 16, dans laquelle le composé tétrapyrrolique est présent à une concentration de 0,1 à 40 μg/ml.

**18.** Utilisation selon la revendication 15, dans laquelle le réactif comprend un premier composant contenant un tampon et un ou plusieurs composés tétrapyrroliques, et un deuxième composant comprenant une entité choisie dans le groupe consistant en une bilirubine oxydase, un agent oxydant, et un sel de diazonium.

**19.** Utilisation selon la revendication 18, dans laquelle le composé tétrapyrrolique est choisi dans le groupe consistant en bilirubine, biliverdine, urobilinogène, et des combinaisons de ceux-ci.

**20.** Utilisation selon la revendication 18 ou la revendication 19, dans laquelle le composé tétrapyrrolique est présent à une concentration de 0,1 à 40 μg/ml.

Figure 1

example 1
example 2
Comparative example 1
Comparative example 2
0.7
0.6
0.5
0.4
0.3
0.2
0.1
0.0
0/5
1/5
2/5
3/5
4/5
5/5
Measured value of direct bilirubin(mg/dl)
Dilution rate

[Figure 2]

0.7
0.6
0.5
0.4
0.3
0.2
0.1
0.0
Measured value of direct bilirubin(mg/dl)
example 3
example 4
Comparative example 3
Comparative example 4
0/5
1/5
2/5
3/5
4/5
5/5
Dilution rate

Figure 3

50
40
30
20
10
0
Measured value of direct bilirubin(mg/dl)
0/10
1/10
2/10
3/10
4/10
5/10
6/10
7/10
8/10
9/10
10/10
Dilution rate

Figure 4

Measured value of direct bilirubin(mg/dl)
40
30
20
10
0
0/10
1/10
2/10
3/10
4/10
5/10
6/10
7/10
8/10
9/10
10/10
Dilution rate

Figure 5

30
20
10
0
Measured value of direct bilirubin(mg/dl)
0/10
1/10
2/10
3/10
4/10
5/10
6/10
7/10
8/10
9/10
10/10
Dilution rate

Figure 6

40
30
20
10
0
0/10
1/10
2/10
3/10
4/10
5/10
6/10
7/10
8/10
9/10
10/10
Dilution rate

Figure 7

20
10
0
example 21
example 22
example 23
example 24
Measured value of direct bilirubin(mg/dl)
0/10
1/10
2/10
3/10
4/10
5/10
6/10
7/10
8/10
9/10
10/10
Dilution rate

Figure 8

30
20
10
0
example 25
example 26
example 27
example 28
Measured value of direct bilirubin(mg/dl)
0/10
1/10
2/10
3/10
4/10
5/10
6/10
7/10
8/10
9/10
10/10
Dilution rate

Figure 9

30
20
10
0
example 29
example 30
example 31
example 32
Measured value of direct bilirubin(mg/dl)
0/10
1/10
2/10
3/10
4/10
5/10
6/10
7/10
8/10
9/10
10/10
Dilution rate